# FASCICULE DE BREVET EUROPEEN

(11) **EP 2 459 644 B1**
(45) Date de publication et mention de la délivrance du brevet: **24.07.2013**
(21) Numéro de dépôt: 10740586.2
(22) Date de dépôt: 27.07.2010
(51) Int. Cl.: C08L 27/06, F16L 9/12, F16L 11/04, F16L 51/00, C08L 51/00

(54) **UTILISATION D'UNE COMPOSITION DE RESINES PVC POUR LA FABRICATION DE TUBES**
VERWENDUNG EINER PVC-HARZZUSAMMENSETZUNG ZUR HERSTELLUNG VON ROHREN
USE OF A PVC RESIN COMPOSITION TO PREPARE PIPES

(30) Priorité: 28.07.2009 FR 0955272
(43) Date de publication de la demande: 06.06.2012
(73) Titulaire: Kem One, 69007 Lyon (FR)
(72) Inventeur: HARDOUIN-DUPARC, Ludovic, F-51100 Reims (FR); MARCOTTE, Jean-François, F-94100 Maisons-Alfort (FR); SCHNITZLER, Jean-Luc, F-78980 Breval (FR); LYOEN, Frédéric, F-27500 Pont-Audemer (FR); LACROIX, Sophie, 29100 VILLLEURBANNE (FR)
(74) Mandataire: Colombet, Alain André
(86) Numéro de dépôt international: PCT/EP2010/060879
(87) Numéro de publication internationale: WO 2011/012617

(56) Documents cités:
- EP-A1- 0 564 035
- EP-A2- 0 313 507
- DE-A1- 10 121 580
- FR-A- 1 566 442
- DATABASE WPI Week 200112 Thomson Scientific, London, GB; AN 2001-106643 XP002608435 & JP 2000 327879 A (SEKISUI CHEM IND CO LTD) 28 novembre 2000 (2000-11-28)
- DATABASE WPI Week 200033 Thomson Scientific, London, GB; AN 2000-379339 XP002608436 & JP 2000 120942 A (SEKISUI CHEM IND CO LTD) 28 avril 2000 (2000-04-28)

## Description

La présente invention concerne l'utilisation pour la fabrication de tubes destinés au transport de l'eau potable ou des liquides physilogiques une composition comprenant un mélange de résines de polychlorure de vinyle particulières.

Dans le domaine de la distribution de l'eau potable, l'utilisation de tubes en polyéthylène (ci-après PE) est très largement répandue. Il existe également des canalisations réalisées en polypropylène ou en polychlorure de vinyle rigide, ces matériaux présentant l'avantage d'être inertes tant chimiquement que physiquement à l'égard de l'eau potable transportée. Ces canalisations sont de fabrication aisée et peu coûteuse de sorte que leur emploi est largement répandu dans les installations de distribution.

Généralement, des dérivés chlorés sont ajoutés à l'eau afin d'éviter la prolifération de bactéries et de champignons dans les canalisations. La présence au sein de ces canalisations de manière continue de ces dérivés chlorés, présentant des propriétés anti-bactériennes et anti-fongiques, provoque des fissures ou des zones de stress-cracking, après une utilisation prolongée de ces canalisations.

Par conséquent, il est recherché des compositions polymères pour la fabrication de tuyaux ou de tubes destinés au transport de fluide, pouvant être par exemple implantés dans des installations fixes ou mobiles de distribution d'eau potable.

Ainsi, le matériau recherché doit répondre à un cahier des charges très précis. Il doit présenter une bonne tenue en pression. En particulier, la pression nominale maximale requise pour l'irrigation est de 16 bars, et pour le transport d'eau potable, de 10 bars. Comme indiqué ci-dessus, le matériau doit être accepté sur le plan du contact alimentaire par les autorités sanitaires. De ce fait, le matériau doit tolérer la présence continue de dérivés chlorés nécessaire à la désinfection, comme évoqué ci-dessus.

De plus, il doit être résistant aux chocs, doit présenter une bonne tenue à l'écrasement, tout en étant suffisamment souple pour pouvoir être facilement enroulable et déroulable.

Idéalement, le matériau devrait pouvoir s'adapter aux raccords mécaniques existants, notamment utilisés aujourd'hui pour les tùyaux en PE.

Enfin, il doit présenter une bonne stabilité dimensionnelle à température ambiante, c'est-à-dire présenter un point de Vicat élevé.

Ainsi, il est recherché un matériau présentant des caractéristiques physiques similaires au PE déjà utilisé, en terme de résistance et de souplesse, tout en tolérant parfaitement la présence continue de dérivés chlorés.

Le polychlorure de vinyle (ci-après PVC) présente l'avantage de parfaitement supporter la présence continue de composés chlorés, de part sa constitution chimique. Mais, le PVC présente l'inconvénient d'être beaucoup trop rigide pour l'utilisation envisagée. Il présente notamment le désavantage de ne pas être enroulable et déroulable.

Des compositions de PVC comprenant des plastifiants ont été envisagées, afin de rendre le matériau plus souple. Mais, la présence de plastifiants au sein du PVC a tendance à abaisser le point de Vicat, conduisant à une stabilité dimensionnelle du matériau inacceptable pour l'utilisation envisagée.

Le document JP2000/327879 divulgue une composition de résine obtenue par copolymérisation de 95 à 90% en poids de chlorure de vinyle avec 5 à 10% en poids de copolymère acrylique. Cette composition base de résine PVC peut être utilisée pour la réalisation de joints résistants aux impacts ou moulables.
Le document JP2000/120942 divulgue des compositions à base de PVC pour la fabrication de tubes visant une flexibilité analogue à celle du polyéthylène et présentant une bonne résistance au feu. Les tubes sont destinés à être utilisés pour la fabrication de cables electriques.

Il a été trouvé qu'une composition de résines de polychlorure de vinyle (PVC) particulières permettait de résoudre les problèmes posés.

L'invention porte sur l'utilisation pour la fabrication de tubes destinés au transport de l'eau potable ou des liquides physilogiques une composition comprenant :
- de 45 à 85 % en poids par rapport au poids total des polymères présents dans la composition d'une ou plusieurs résines de PVC non greffées présentant une valeur de Kwert comprise entre 50 et 90,de préférence compris entre 60 et 80, et
- de 15 à 55% en poids par rapport au poids total des polymères présents dans la composition d'une ou plusieurs résines de PVC greffées sur un ou plusieurs polymères choisis parmi les polyacrylates d'alkyle en C1-C18, les polyméthacrylates d'alkyle en C1-C18 et leur mélange.

Par polymères présents dans la composition, on entend au sens de la présente invention la ou les résines de PVC non greffées et la ou les résines de PVC greffée.

Il est précisé que l'expression "compris entre" doit s'entendre comme incluant chacune des bornes mentionnées.

### Le po/ychlorure de vinyle non greffé

La composition comprend de 45 à 85 % en poids par rapport au poids total des polymères présents dans la composition d'une ou plusieurs résines de PVC non greffée présentant une valeur de Kwert comprise entre 50 et 90. De préférence, la valeur de la constante Kwert est comprise entre 60 et 80.

La valeur de Kwcrt est mesurée selon une méthode connue. L'indice de viscosité évalué selon la norme ISO R 174 est mesuré à 25°C à une concentration de 0,5g de PVC dans 100mL de cyclohexanone. A l'indice de viscosité obtenue correspond une valeur de Kwert donnée selon la norme DIN 53726.

Selon un mode de réalisation, la résine de PVC non greffée est obtenue par un procédé en suspension ou en micro-suspension. Du PVC préparé selon une méthode en émulsion ou en masse peut aussi être utilisé.

De préférence, la résine de PVC non greffée est linéaire et non réticulée.

De préférence, la composition utilisée dans l'invention comprend de 65 à 80 % en poids par rapport au poids total des polymères présents dans la composition d'une ou plusieurs résines de PVC non greffées présentant une valeur de Kwert comprise entre 50 et 90.

### Le polychlorure de vinyle greffé

La composition comprend de 15 à 55% en poids par rapport au poids total des polymères présents dans la composition d'une ou plusieurs résines de PVC greffées sur un ou plusieurs polymères choisi parmi les polyacrylates d'alkyle en C1-C18, les polyméthacrylatcs d'alkyle en C1-C18 et leur mélange, également désigné par poly(meth)acrylate d'alkyle en C1-C18.

Plus particulièrement, la résine PVC greffée est un élastomère thermoplastique à base de copolymérisats greffés de chlorure de vinyle, contenant de 35 à 60% en poids, par rapport au poids total du copolymérisat greffé de chlorure de vinyle, d'un (co)polymère greffé.

Par « élastomère », on entend au sens de la présente invention un polymère ou co-polymère présentant une température de transition vitreuse inférieure ou égale à 0°C. Un tel polymère ou co-polymère présente de préférence un module de Young, mesuré à la température d'utilisation, compris entre 10 000 Pa et 100 000 000 Pa, et, de préférence, compris entre 50 000 Pa et 10 000 000 Pa.

La résine PVC greffée peut être préparée par (co)polymérisation :
- de 80 à 100% en poids de chlorure de vinyle ainsi que
- de 0 à 20% en poids de comonomères supplémentaires copolymérisables, éthyléniquement insaturés, lors d'une polymérisation de 0 à 45°C, et
- de 40 à 65% en poids, par rapport au poids total du copolymérisat greffé de chlorure de vinyle, d'une base greffée réticulée contenant un réseau copolymérisat comportant des motifs choisis parmi les esters d'acide acrylique, les esters d'acide méthacrylique et leur mélange, et éventuellement ayant de 0,01 à 5% en poids d'unités comonomères supplémentaires copolymérisables, éthyléniquement avec des esters d'acide acrylique et/ou méthacrylique.

De préférence, les esters d'acide acrylique et méthacrylique sont des esters d'alcool comportant 1 à 18 atomes de carbones. Les acrylates et méthacrylates de méthyle, éthylc, propyle, n-butyle, d'hexyle, d'heptyle, d'octyle, de 2-éthylhexyle, de nonyle, d'isononyle, de décyle, de isodécyle, de undécyle, de dodécyle et de tridécylc sont préférés.

De préférence, les acrylates d'alkyle en C2-C16 et les méthacrylates d'alkyle en C6-C13 sont préférés.

Plus particulièrement, l'acrylate de n-butyle, l'acrylate de 2-éthylhcxylc et leurs mélanges sont préférés.

De préférence, l'élastomère comporte de 45 à 60% en poids, par rapport au poids total du copolymérisat greffé, d'une base greffée réticulée, se composant d'un copolymérisat réticulé d'acrylate de n-butyle, d'acrylate de 2-éthylhexyle et leurs mélanges, ou d'un copolymérisat réticulé d'ester d'acide acrylique/éthylène/acétate de vinyle, ayant une teneur en acrylate compris entre 35 et 70% en poids ou des mélanges des copolymérisats cités, les copolymérisats étant réticulés avec de 0,05 à 0,5% en poids d'un ou de plusieurs comonomères copolymérisables plusieurs fois éthyléniquement insaturés provenant du groupe de phtalate de diallyle, du méthacrylate d'allyle, du diméthacrylate d'éthylèneglycole, du diacrylate de butylèneglycole, du diacrylate de triméthylèneglycole, du triacrylate de triméthyolpropane.

Plus particulièrement, la composition comprend une teneur en base greffée réticulée contenant un réseau copolymérisat comportant des motifs choisi parmi les esters d'acide acrylique, les esters d'acide méthacrylique et leur mélange, comprise entre 15 et 98% en poids par rapport au poids total des polymères présents dans la composition, et de préférence entre 40 et 60%.

De préférence, la résine VK710 commercialisée par la société Vinnolit est utilisée.

Selon un autre mode de réalisation de l'invention, la résine PVC greffée peut être préparée par copolymérisation de chlorure de vinyle et de le poly méthylméthacrylate-b-acrylate de butyle-b-méthyl méthacrylate (MAM), des polymères ou copolymères acryliques souples comme les résines à base d'esters acryliques telles que le polyacrylate de butyle et ses copolymères avec d'autres monomères acryliques ou vinyliques, ainsi que leurs mélanges.

Les élastomères peuvent notamment être choisis parmi les dérivés acryliques commercialisés par la société ARKEMA sous les dénominations commerciales Durastrength® 200, 320, 340 et 360, les dérivés acryliques commercialisés par la société Rohm & Haas sous les dénominations commerciales Paraloid® KM342, KM342B, KM361, KM370 et KM1, les dérivés acryliques commercialisés par la société KANEKA sous les dénominations commerciales FM22® et FM 50®.

De préférence, la composition utilisée dans l'invention comprend de 20 à 45% en poids par rapport au poids total des polymères présents dans la composition d'une ou plusieurs résines de PVC greffées telles que définies ci-dessus.

De préférence, la composition comprend de 10 à 20% en poids par rapport au poids total des polymères présents dans la composition de poly(meth)acrylate d'alkyle en C1-C18 présent dans la résine PVC greffée.

Plus particulièrement, la composition comprend de 12 à 18% en poids par rapport au poids total des polymères présents dans la composition de poly(meth)acrylate d'alkyle en C1-C18 présent dans la résine PVC greffée.

Selon un autre mode de réalisation, la composition peut comprendre un polymère compatibilisant choisi parmi les copolymères ou terpolymères de chlorure de vinyle et acétate de vinyle (VC/VA), les copolymères ou terpolymères de chlorure de vinyle et de dérivés d'acrylique (VC/DA), les polyuréthanes thermoplastiques (TPU), les polyétheresters thermoplastiques, les copolymères élastomères acrylonitrile/butadiène (NBR), les copolymères éthylène/monomère vinylique (EVA), les terpolymèrcs éthylène/monomère vinylique/carbonyle, les élastomères acryliques processables à l'état fondu, les copolymères à blocs polyamide et à blocs polyéther ou polyéther bloc amides, les polyéthylènes chlorés ou chlorosulfonés, les polymères éthylène/(méth)acrylate d'alkyle ou acide (méth)acrylique fonctionnalisés ou non, les polymères core-shell type MBS, les terpolymères bloc SBM, le PVDF et les résines polyamides en poudre.

Un exemple de copolymère VC/VA est le produit Lacovyl GA de Arkema, un exemple de copolymère VC/DA est le produit Vinnolit VK de Vinnolit, un exemple de TPU est le produit Estane de Goodrich, un exemple de polyétherester thermoplastique est le produit Hytrel de DuPont, un exemple de copolymère acrylonitrile / butadiène (NBR) est le produit Chemigum d'Eliokem, un exemple de polyéther bloc amidc est le produit Pebax de Arkema, un exemple d'EVA est le produit Evatane d'Arkema, un exemple de terpolymère éthylène/ monomère vinylique/carbonyle est le produit Elvaloy de DuPont, des exemples de polymères éthylène/(méth)acrylate d'alkyle ou acide (méth)acrylique fonctionnalisés ou non sont les produits Lotryl, Lotader et Orevac de Arkema, un exemple de polyéthylènes chlorés ou chlorosulfonés est le produit Tyrin de DuPont, un exemple d'élastomère acrylique processable à l'état fondu est le produit Alcryn de Apa, un exemple de résines polyamides en poudre est le produit Orgasol de Arkema.

La composition peut également comprendre entre autres, un additif pouvant notamment être choisi parmi des pigments ou charges, des stabilisants, des anti-oxydants, des additifs de mise en oeuvre, des lubrifiants ou des agents ignifugeants. En particulier, parmi les additifs communément utilisés dans des compositions à base de résine vinylique, on peut citer des sels métalliques d'acide carboxylique organique, des acides phosphoriques organiques, des zéolites, des hydrotalcites, des composés époxydés, des béta-dicétones, des alcools polyhydriques, des antioxydants phosphorés, soufrés ou phénoliques, des absorbeurs ultraviolets par exemple des benzophénoncs, benzotriazoles, et dérivés d'oxanilide, des cyanoacrylates, des stabilisants lumière à amines encombrées de type HALS (hindered amine light stabilizer), des sels d'acide perchlorique, et d'autres composés inorganiques à base de métaux, des lubrifiants par exemple des cires organiques, des alcools gras, des acides gras, des esters gras, des sels métalliques, des charges par exemple de la craie ou du talc, et des pigments tels que le noir de carbone, les phtalocyanines de cuivre.

De plus, la composition peut comprendre, en poids par rapport au poids total de la composition 0,1 à 10 % des additifs mentionnés ci-dessus.

La composition peut être utilisé(e) pour constituer une structure. Cette structure peut être monocouche, lorsqu'elle n'est formée que de la composition.

Cette structure peut également être une structure multicouche, lorsqu'elle comprend au moins deux couches et que l'une au moins des différentes couches formant la structure est formée de la composition.

De préférence, la composition constitue une structure monocouche.

La structure, qu'elle soit monocouche ou multicouche, peut notamment se présenter sous la forme de fibres, d'un film, d'un tube, d'un corps creux, d'une pièce injectée ou encore d'une lentille. La composition peut être préparée par toute méthode, qui rend possible l'obtention d'un mélange homogène contenant la composition selon l'invention, et éventuellement d'autres additifs, telle que l'extrusion à l'état fondu, le compactage ou encore le malaxage, utilisant par exemple un malaxeur à rouleau (mélangeur à cylindres) ou un mélangeur interne.

Plus particulièrement, la composition est préparée par mélange à l'état fondu de tous ses composants, notamment dans un procédé dit en direct.

Avantageusement, la composition peut être obtenue sous forme de granulés par compoundage sur un outil connu de l'homme de l'art tel que : extrudeuse mono-vis ou bi vis, comalaxeur, mélangeur interne.

La composition obtenue par le procédé de préparation décrit ci-dessus peut être ensuite transformée pour une utilisation ou une transformation ultérieure connue par l'homme de l'art à l'aide d'outils tels que : presse à injecter, extrudeuse, etc.

Le procédé de préparation de la composition peut aussi utiliser une extrudeuse mono-vis ou bi-vis alimentant, sans granulation intermédiaire, une presse à injecter ou une extrudeuse selon un dispositif de mise en oeuvre connu par l'homme de l'art.

Selon un mode de réalisation particulier, le tube est fabriqué selon un procédé de fabrication en continu avec étirage bi-axial, ledit procédé est décrit dans la demande de brevet française FR 2 806 956.

La composition selon l'invention est utilisé(e) pour la fabrication de tubes ou tuyaux pour des applications dans le domaine de la distribution d'eau potable, comme par exemple des canalisations, ou bien des tuyaux d'arrosage, dans le domaine médical ou paramédical, comme par exemple les tuyaux utilisés pour le transport de liquides physiologiques.

### EXEMPLES

### 1/Préparation des compositions

Les compositions suivantes ont été préparées à partir des produits suivants :
Le polychlorure de vinyle :
   Résine PVC : polychlorure de vinyle de KWert de 70 commercialisé par la société Arkema sous la dénomination Lacovyl S7015.
Le polychlorure de vinyle greffé :
   Résine PVC greffé : polychlorure de vinyle greffé à 50% sur un polyacrylate commercialisé par la société Vinnolit sous la dénomination VK710.
Les plastifiants :
   Plastifiant DOP commercialisé par le société Arkema sous la dénomination Garbeflex.
Les stabilisants :
   Huile de soja commercialisée par la société Akcros sous la dénomination Lankroflex.
   Ba/Zn : mélange de barium et de zinc commercialisé par la société Lagor sous la dénomination Lastab.
   Ca/Zn : mélange de calcium et de zinc commercialisé par la société Reagens sous la dénomination Stabiol.
   Stéarate de Ca : stéarate de calcium commercialisé par la société Greven sous la dénomination Ligastab CAPSE.
   Stéarate de Zn : stéarate de zinc commercialisé par la société Greven sous la dénomination Ligastab ZNE.
Les additifs :
   Processing Aid : agent de mise en oeuvre (en langue anglaise processing aid) constitué de poly méthacrylate de méthyle (PMMA) commercialisé par la société Arkema sous la dénomination Plastistrenght.
   Lubrifiant externe : cire de polyéthylène commercialisée par la société Honeywell sous la dénomination AC wax.
   Lubrifiant interne : cire de polyéthylène commercialisée par la société Oleon sous la dénomination Radia
   Antioxydant : phosphite commercialisé par la société Baerlocher sous la dénomination Barostab
   Azurant : violet outremer commercialisé par la société Holliday sous la dénomination Prestige

Selon les proportions du tableau suivant :

| Compositions | A Compa | B Compa | 1 inv |
|---|---|---|---|
| Résine PVC | 100 | 34 | 70 |
| Résine PVC greffé | - | 66 | 30 |
| Plastifiant DOP | 28 | - | - |
| Stabilisant Huile de soja | 4 | 7,5 | 7,5 |
| Stabilisant Ba/Zn | 2 | 4 | - |
| Stabilisant Ca/Zn | - | - | 4 |
| Stabilisant Stéarate Ca | - | 0,4 | 0,4 |
| Stabilisant Stéarate Zn | - | - | 0,2 |
| Proc. Aid | - | 1 | 1 |
| Lubrifiant interne | - | - | 0,5 |
| Lubrifiant externe | 0,2 | - | 0,6 |
| Antioxydant | 0,6 | - | 0,6 |
| Azurant | 0,02 | - | 0,6 |

Les compositions A et B sont des compositions comparatives. La composition 1 est une composition utilisée dans l'invention.

Les compositions de cette étude sont préparées par mélange (compoundage) à l'aide d'une extrudeuse monovis Samafor diamètre 60 mm L/D = 28. Les ingrédients sont introduits via la trémie d'alimentation en 1^{er} fourreau.

### 2/ Extrusion des compositions

Les différentes compositions sont extrudées à une température d'extrusion comprise entre 160 et 180°C.

### 3/Caractérisation des matériaux

### 3.1 Mesure du point de Vicat (°C) : Le point Vicat est évalué selon la norme ISO 306.

### 3.2 Mesure de la dureté (Shore D): La dureté est évaluée selon la norme ISO 868.

### 3.3 Rigidité annulaire du tube (kN/m²) : La rigidité annulaire est évaluée selon la norme ISO 9969.

### 3.4 Mesure de la résistance maximale (MPa) : La résistance maximale est évaluée selon la norme ISO 527.

Des tubes des compositions précédentes sont comparés à un tube en PE. Les résultats sont reportés dans le tableau ci-dessous :

| Compositions | A Compa | B Compa | 1 Inv | PE Compa |
|---|---|---|---|---|
| Point Vicat 5 kg (°C) | 42 | 48 | 62 | 70 |
| Dureté (Shore D) | 44 | 51 | 64 | 57 |
| Rigidité (Shore D) | 11 | 52 | 183 | 52 |
| R max (Shore D) | 21 | 20 | 30 | 13 |

### 3.5 Mesure du pourcentage de l'allongement à la rupture en milieu chloré (MPa) :

Pour cette étude, trois tubes sont comparés :
- le premier tube est en polyéthylène haute densité (PE, tube NF PE 80 pipelife 32×3,0 PN 12,5),
- le second tube est en polychlorure de vinyle (PVC, tube NF PVC Alphacan Lucoflex 32×2,4 PN 16) et
- le dernier tube, noté 2 (inv) est un tube selon l'invention monocouche comportant un mélange de PVC (homopolymère à une teneur de 70% dans le mélange, PVC type S110P) et de PVC greffé acrylate de butyle (30%, VK 710). Ainsi, la teneur en monomère acrylique de cette composition est de 15%.

Ces tubes sont placés pendant 3 mois à 40°C dans une eau désinfectée au dioxyde de chlore (ClO₂) en une teneur de 100ppm. Cette teneur est très supérieure à celle contenue dans les canalisations d'eau potable standart. Pour les traitements standarts, la teneur en ClO₂ est de 2 ppm à température ambiante souterraine.

L'allongement à la rupture est mesuré avant immersion (t = 0) et après 3 mois d'immersion (t = 3 mois).

L'oxydation des polymères conduit généralement à des coupures de chaînes. Les coupures de chaînes entraînent une diminution de l'allongement à la rupture en traction. Les tests de traction on été réalisés selon la norme ISO 527, dans des éprouvettes 1Ba. La vitesse de traction est de 15mm/min. Le tableau ci-dessous donne les résultats obtenus des pourcentages en allongement à la rupture obtenus :

| Allongement (%) | PE Compa | PVC Compa | 2 Inv |
|---|---|---|---|
| At=0 | 500 | 125 | 130 |
| At = 3 mois | 100 | 140 | 140 |

L'allongement à la rupture du tube en PE s'effondre après les 3 mois d'immersion dans l'eau chlorée.

### 4/Conclusion

La composition utilisée dans l'invention présente l'avantage d'être compatible avec la présence continue de composés chlorés, d'être résistante à la pression (Rmax), tout en étant suffisamment souple pour être déroulable.

## Revendications

1. Utilisation pour la fabrication de tubes destinés au transport de l'eau potable ou de liquides physiologiques d'une composition comprenant :
- de 45 à 85 % en poids par rapport au poids total des polymères présents dans la composition d'une ou plusieurs résines de PVC non greffées présentant une valeur de Kwert comprise entre 50 et 90, mesuré à 25°C à une concentration de 0,5 g de PVC dans 100 mL de cyclohexanone selon les normes ISO R 174 et DIN 53726, et
- de 15 à 55 % en poids par rapport au poids total des polymères présents dans la composition d'une ou plusieurs résines de PVC greffées sur un ou plusieurs polymères choisis parmi les polyacrylates d'alkyle en C₁-C₁₈, les polyméthacrylates d'alkyle en C₁-C₁₈ et leur mélange.

2. Utilisation selon la revendication 1, **caractérisée en ce que** la résine de PVC non greffée présente dans la composition est linéaire et non réticulée.

3. Utilisation selon la revendication 1 ou 2, **caractérisée en ce que** la résine de PVC non greffée présente dans la composition présente une valeur de KWert comprise entre 60 et 80, mesuré à 25°C à une concentration de 0,5 g de PVC dans 100 mL de cyclohexanone selon les normes ISO R 174 et DIN 53726.

4. Utilisation selon l'une quelconque des revendications précédentes, **caractérisée en ce que** la composition comprend de 10 à 20% en poids, par rapport au poids total des polymères présents dans la composition, de poly(meth)acrylate d'alkyle en C₁-C₁₈ présent dans la résine PVC greffée.

5. Utilisation selon l'une quelconque des revendications précédentes, **caractérisée en ce que** la résine de PVC greffée comprend en tant que monomères acrylates : de l'acrylate de n-butyle, l'acrylate de 2-éthylhexyle et leurs mélanges.

6. Utilisation selon l'une quelconque des revendications précédentes, **caractérisée en ce que** la composition comprend au moins un additif choisi parmi des sels métalliques d'acide carboxylique organique, des acides phosphorique organique, des zéolites, des hydrotalcites, des composés époxidés, des béta-dicétones, des alcools polyhydriques, des antioxydants phosphores, soufrés ou phénolique, des absorbeurs ultraviolet, des cyanoacrylates, des stabilisants lumière à amines encombrées de type HALS (hindered amine light stabilizer), des sels d'acide perchlorique, et d'autres composés inorganiques à base de métaux, des lubrifiants, des sels métalliques, des charges et des pigments.

7. Utilisation selon l'une quelconque des revendications précédentes, **caractérisée en ce que** la composition se trouve sous forme d'une structure monocouche.

8. Utilisation selon l'une quelconque des revendications précédentes, **caractérisée en ce que** la composition se présente sous la forme de fibres, d'un film, de feuille, d'un tube, d'un corps creux ou d'une pièce injectée.

## Patentansprüche

1. Verwendung einer Zusammensetzung zur Herstellung von Rohren, die für den Transport von Trinkwasser oder von physiologischen Flüssigkeiten bestimmt sind, umfassend:
- im Verhältnis zum Gesamtgewicht der in der Zusammensetzung vorhandenen Polymere 45 Gew.-% bis 85 Gew.-% von einem oder mehreren nicht gepfropften PVC-Harzen, die bei 25°C bei einer Konzentration von 0,5 g PVC in 100 ml Cyclohexanon gemäß den Normen ISO R 174 und DIN 53726 gemessen einen K-Wert von zwischen 50 und 90 haben, und
- im Verhältnis zum Gesamtgewicht der in der Zusammensetzung vorhandenen Polymere 15 Gew.-% bis 55 Gew.-% von einem oder mehreren PVC-Harzen, die auf ein oder mehrere Polymere gepfropft sind, die aus C1-C18-Alkylpolyacrylaten, C1-C18-Alkylpolymethacrylaten und deren Mischung ausgewählt sind.

2. Verwendung gemäß Anspruch 1, **dadurch gekennzeichnet, dass** das in der Zusammensetzung vorhandene nicht-gepfropfte PVC-Harz linear und nicht netzförmig ist.

3. Verwendung gemäß Anspruch 1 oder 2, **dadurch gekennzeichnet, dass** das in der Zusammensetzung vorhandene nicht-gepfropfte PVC-Harz bei 25°C bei einer Konzentration von 0,5 g PVC in 100 ml Cyclohexanon gemäß den Normen ISO R 174 und DIN 53726 gemessen einen K-Wert zwischen 60 und 80 hat.

4. Verwendung gemäß irgendeinem der vorhergehenden Ansprüche, **dadurch gekennzeichnet, dass** die Zusammensetzung im Verhältnis zum Gesamtgewicht der in der Zusammensetzung vorhandenen Polymere 10 Gew.-% bis 20 Gew.-% C1-C18-Alkylpoly(meth)acrylat umfasst, das sich in dem gepfropften PVC-Harz befindet.

5. Verwendung gemäß irgendeinem der vorhergehenden Ansprüche, **dadurch gekennzeichnet, dass** das gepfropfte PVC-Harz als Acrylatmonomere n-Butylacrylat, 2-Ethylhexylacrylat und deren Mischungen umfasst.

6. Verwendung gemäß irgendeinem der vorhergehenden Ansprüche, **dadurch gekennzeichnet, dass** die Zusammensetzung mindestens ein Additiv umfasst, das aus Metallsalzen der organischen Carbonsäure, organischen Phosphorsäuren, Zeoliten, Hydrotalciten, epoxidierten Verbindungen, Betadiketonen, mehrwertigen Alkoholen, Phosphor-, Schwefel- oder phenolischen Antioxidantien, UV-Absorbern, Cyanoacrylaten, Lichtstabilisatoren mit gehinderten Aminen des HALS-Typs (engl. Hindered Amine Light Stabilizer), Salzen der Perchlorsäure und anderen anorganischen Verbindungen auf Basis von Metallen, Schmiermitteln, Metallsalzen, Füllstoffen und Pigmenten ausgewählt ist.

7. Verwendung gemäß irgendeinem der vorhergeherden Ansprüche, **dadurch gekennzeichnet, dass** die Zusammensetzung in Form einer einschichtigen Struktur vorgesehen ist.

8. Verwendung gemäß irgendeinem der vorhergehenden Ansprüche, **dadurch gekennzeichnet, dass** die Zusammensetzung in Form von Fasern, eines Films, eines Blatts, eines Rohrs, eines hohlen Körpers oder eines Spritzgussteils vorgesehen ist.

## Claims

1. Use of a composition for the production of tubes intended for the transport of drinking water or physiological fluids, said composition comprising:
• 45% to 85% by weight in relation to the total weight of the polymers present in the composition of one or more non-grafted PVC resins with a K value ranging between 50 and 90 measured at 25°C with a concentration of 0.5 g of PVC in 100 mL of cyclohexanone in accordance with standards ISO R 174 and DIN 53726, and
• 15% to 55% by weight in relation to the total weight of the polymers present in the composition of one or more PVC resins grafted onto one or more polymers selected from C₁-C₁₈ alkyl polyacrylates, C₁-C₁₈ alkyl polymethacrylates and mixture thereof.

2. Use according to claim 1, **characterised in that** the non-grafted PVC resin present in the composition is linear and non-cross-linked.

3. Use according to claim 1 or 2, **characterised in that** the non-grafted PVC resin present in the composition has a K value ranging between 60 and 80 measured at 25°C with a concentration of 0.5 g of PVC in 100 mL of cyclohexanone in accordance with standards ISO R 174 and DIN 53726.

4. Use according to any one of the preceding claims, **characterised in that** the composition comprises 10% to 20% by weight in relation to the total weight of the polymers present in the composition of C₁-C₁₈ alkyl poly(meth)acrylate present in the grafted PVC resin.

5. Use according to any one of the preceding claims, **characterised in that** the grafted PVC resin comprises n-butyl acrylate, 2-ethylhexyl acrylate and mixtures thereof as acrylate monomers.

6. Use according to any one of the preceding claims, **characterised in that** the composition comprises at least one additive selected from the metal salts of organic carboxylic acid, organic phosphoric acids, zeolites, hydrotalcites, epoxy compounds, beta-diketones, polyhydric alcohols, sulphurous or phenolic phosphorus antioxidants, ultraviolet absorbers, cyanoacrylates, hindered amine light stabilisers (HALS), perchloric acid salts, and other metal-based inorganic compounds, lubricants, metal salts, fillers and pigments.

7. Use according to any one of the preceding claims, **characterised in that** composition is in the form of a single-layer structure.

8. Use according to any one of the preceding claims, **characterised in that** the composition is provided in the form of fibres, a film, a sheet, a tube, a hollow body or an injection-moulded part.
